# EUROPEAN PATENT APPLICATION

(11) **EP 4 537 903 A1**
(43) Date of publication of application: **16.04.2025**
(21) Application number: 23819821.2
(22) Date of filing: 06.06.2023
(51) Int. Cl.: A61P 31/12, C09D 5/14, C09D 139/06, A61K 9/08, A61K 47/10, A61K 47/32, C09D 7/20, C09D 7/62, C09D 7/63, A61K 33/06, A61K 33/34

(54) **ANTIVIRAL AEROSOL COMPOSITION, COATING FILM, AND ARTICLE**

(30) Priority: 08.06.2022 JP 2022093309
(71) Applicant: Toyo Seikan Group Holdings, Ltd., Shinagawa-ku Tokyo 141-8627 (JP); Toyo Aerosol Industry Co., Ltd., Tokyo 141-0022 (JP)
(72) Inventor: KIMURA Mariko, Yokohama-shi, Kanagawa 240-0062 (JP); OHASHI Kazuaki, Yokohama-shi, Kanagawa 240-0062 (JP); NISHIKATA Yuri, Tokyo 141-0022 (JP)
(74) Representative: Körfer, Thomas
(86) International application number: PCT/JP2023/020921
(87) International publication number: WO 2023/238841

(57) **Abstract**

An object of the present invention is to provide an antiviral aerosol composition that has high stability and can be aerosol-applied without causing nozzle clogging or the like, the antiviral aerosol composition being capable of forming a coating film whose copper-doped silica exhibits excellent antiviral properties and which is firmly retained on the surface of an article; a coating film formed from the antiviral aerosol composition; and an article comprising the coating film. The antiviral aerosol composition of the present invention as a means for resolution comprises (1) a copper-doped silica, (2) a vinylpyrrolidone unit-containing polymer, (3) an alcohol-based solvent or a mixed solvent of an alcohol-based solvent and water, and (4) a propellant. The coating film of the present invention is formed from the antiviral aerosol composition of the present invention. The article of the present invention comprises the coating film of the present invention.

## Description

### Technical Field

The present invention relates to an antiviral aerosol composition, a coating film, and an article.

### Background Art

As is well known, in recent years, with the emergence of new strains of influenza virus and coronavirus that cause severe symptoms upon infection, etc., there has been a growing interest in antiviral agents to combat viral infections. Viruses can be broadly classified, based on their structure, into viruses with envelopes composed of lipids or glycoproteins (enveloped viruses) and viruses without envelopes (non-enveloped viruses). Influenza virus is a typical example of an enveloped virus, while norovirus is a typical example of a non-enveloped virus. Compared to enveloped viruses, non-enveloped viruses are said to be more resistant to alcohol and heat, and be more infectious. For this reason, research and development of antiviral agents that exhibit excellent antiviral action not only against enveloped viruses but also against non-enveloped viruses has been actively conducted, and the present inventors have also proposed, in Patent Document 1, an inorganic antiviral agent composed of a copper-doped silica as an antiviral agent that exhibits excellent antiviral action not only against enveloped viruses but also against non-enveloped viruses.

The inorganic antiviral agent proposed by the present inventors in Patent Document 1 can be utilized as a material for imparting antiviral properties to various articles around us. However, when its mode of use employed is a mode in which the copper-doped silica is incorporated into an aerosol composition, and the composition is aerosol-applied to the surface of an article, it is necessary that a coating film formed from the copper-doped silica-containing aerosol composition is a coating film whose copper-doped silica exhibits excellent antiviral properties and which is firmly retained on the surface of an article, and it is also necessary that the copper-doped silica-containing aerosol composition has high stability and can be aerosol-applied without causing nozzle clogging or the like.

### Prior Art Documents

Patent Document

Patent Document 1: JP2021-175741A

### Summary of the Invention

### Problems that the Invention is to Solve

Thus, an object of the present invention is to provide an antiviral aerosol composition that has high stability and can be aerosol-applied without causing nozzle clogging or the like, the antiviral aerosol composition being capable of forming a coating film whose copper-doped silica exhibits excellent antiviral properties and which is firmly retained on the surface of an article; a coating film formed from the antiviral aerosol composition; and an article comprising the coating film.

### Means for Solving the Problem

The present inventors have conducted extensive research in view of the above points. As a result, they have found that in the production of an aerosol composition containing a copper-doped silica, when a vinylpyrrolidone unit-containing polymer is used as a resin component (film-forming agent), a coating film formed from the aerosol composition is a coating film whose copper-doped silica exhibits excellent antiviral properties and which is firmly retained on the surface of an article, and also found that the aerosol composition has high stability and can be aerosol-applied without causing nozzle clogging or the like.

The antiviral aerosol composition of the present invention accomplished based on the above findings comprises, as defined in claim 1:
(1) a copper-doped silica;
(2) a vinylpyrrolidone unit-containing polymer;
(3) an alcohol-based solvent or a mixed solvent of an alcohol-based solvent and water; and
(4) a propellant.

In addition, the antiviral aerosol composition according to claim 2 is the antiviral aerosol composition according to claim 1, wherein the silica is further doped with aluminum.

In addition, the antiviral aerosol composition according to claim 3 is the antiviral aerosol composition according to claim 1, wherein the silica is a porous silica.

In addition, the antiviral aerosol composition according to claim 4 is the antiviral aerosol composition according to claim 1, wherein the pyrrolidone unit-containing polymer is a copolymer of a vinylpyrrolidone unit and a non-vinylpyrrolidone unit.

In addition, the antiviral aerosol composition according to claim 5 is the antiviral aerosol composition according to claim 4, wherein the ratio between the vinylpyrrolidone unit and the non-vinylpyrrolidone unit (vinylpyrrolidone unit:non-vinylpyrrolidone unit) is 2:8 to 8:2.

In addition, the antiviral aerosol composition according to claim 6 is the antiviral aerosol composition according to claim 4, wherein the copolymer of a vinylpyrrolidone unit and a non-vinylpyrrolidone unit is a copolymer of vinylpyrrolidone and vinyl acetate.

In addition, the antiviral aerosol composition according to claim 7 is the antiviral aerosol composition according to claim 1, wherein the pyrrolidone unit-containing polymer is polyvinylpyrrolidone.

In addition, the antiviral aerosol composition according to claim 8 is the antiviral aerosol composition according to claim 1, wherein the pyrrolidone unit-containing polymer has a weight average molecular weight of 5,000 to 1,500,000.

In addition, the antiviral aerosol composition according to claim 9 is the antiviral aerosol composition according to claim 1, wherein the alcohol-based solvent is ethanol.

In addition, the antiviral aerosol composition according to claim 10 is the antiviral aerosol composition according to claim 1, wherein the propellant is at least one member selected from the group consisting of a liquefied petroleum gas, a nitrogen gas, a carbon dioxide gas, dimethyl ether, a hydrofluoroolefin, and a hydrofluorocarbon.

In addition, the antiviral aerosol composition according to claim 11 is the antiviral aerosol composition according to claim 1, wherein the contents of the copper-doped silica, the vinylpyrrolidone unit-containing polymer, the alcohol-based solvent or the mixed solvent of an alcohol-based solvent and water, and the propellant in the antiviral aerosol composition, respectively, are as follows: the content of the copper-doped silica is 0.1 to 5 wt%, the content of the vinylpyrrolidone unit-containing polymer is 0.1 to 5 wt%, the content of the alcohol-based solvent or the mixed solvent of an alcohol-based solvent and water is 30 to 90 wt%, and the content of the propellant is 5 to 65 wt%.

In addition, the antiviral aerosol composition according to claim 12 is the antiviral aerosol composition according to claim 1, wherein the weight ratio between the copper-doped silica and the vinylpyrrolidone unit-containing polymer (copper-doped silica:vinylpyrrolidone unit-containing polymer) is 5:95 to 75:25.

In addition, the method for producing an antiviral aerosol composition of the present invention comprises, as defined in claim 13, a step of mixing a stock solution with (4) a propellant,
the stock solution being obtained by a preparation method comprising a step in which
(1) a copper-doped silica;
(2) a vinylpyrrolidone unit-containing polymer; and
(3) an alcohol-based solvent or a mixed solvent of an alcohol-based solvent and water are housed in a treatment container and ball-milled.

In addition, the coating film of the present invention is, as defined in claim 14, formed from the antiviral aerosol composition according to claim 1.

In addition, the article of the present invention comprises, as defined in claim 15, the coating film according to claim 14.

### Effect of the Invention

The present invention makes it possible to provide an antiviral aerosol composition that has high stability and can be aerosol-applied without causing nozzle clogging or the like, the antiviral aerosol composition being capable of forming a coating film whose copper-doped silica exhibits excellent antiviral properties and which is firmly retained on the surface of an article; a coating film formed from the antiviral aerosol composition; and an article comprising the coating film.

### Mode for Carrying Out the Invention

The antiviral aerosol composition of the present invention comprises:
(1) a copper-doped silica;
(2) a vinylpyrrolidone unit-containing polymer;
(3) an alcohol-based solvent or a mixed solvent of an alcohol-based solvent and water; and
(4) a propellant.

In the antiviral aerosol composition of the present invention, the copper-doped silica may be the inorganic antiviral agent described in Patent Document 1, for example. Here, "copper-doped silica" means silica in which copper is incorporated through chemical bonding into an inorganic network composed of silica-forming siloxane bonds. The copper-doped silica may be porous or non-porous, and its shape is not particularly limited.

The copper-doped silica may be further doped with a metal such as aluminum, zirconium, cobalt, manganese, or iron.

The content of copper in the copper-doped silica (in the case of using an additional metal in combination with copper, their total amount) is, for example, 0.01 to 40 wt%, preferably 0.1 to 20 wt%, and more preferably 1 to 10 wt%. When the content of copper in the copper-doped silica is less than 0.01 wt%, sufficient antiviral action may not be obtained, while silica doped with copper in an amount of more than 40 wt% may be difficult to produce. In the case of using an additional metal in combination with copper, the content ratio between cupper and the additional metal may be such that the content of the additional metal is 0.1 to 2 times the content of copper, for example.

In the case where the copper-doped silica is porous, the copper-doped porous silica may be the one described by the present inventors in JP2020-15640A, for example. The specific description is as follows.

As a porous silica, for example, a mesoporous silica in which fine pores (mesopores) having a diameter of 2 to 50 nm are regularly arranged can be mentioned.

It is preferable that the porous silica has a specific surface area of 500 to 2000 m²/g, for example, for the reason that durability can be maintained.

The production of a copper-doped mesoporous silica can be performed according to the following method known per se described in JP2020-15640A, for example.

### (Step 1)

First, a surfactant and a raw material for doping copper in a mesoporous silica are dissolved in a solvent and stirred at 30 to 200°C for 0.5 to 10 hours, for example, thereby forming micelles of the surfactant.

The amount of the surfactant dissolved in the solvent is, for example, 10 to 400 mmol/L, preferably 50 to 150 mmol/L. Alternatively, the amount of the surfactant dissolved in the solvent is, per 1 mol of a silica raw material added in the below-described Step 2, for example, 0.01 to 5.0 mol, preferably 0.05 to 1.0 mol.

As the surfactant, any of cationic surfactants, anionic surfactants, and nonionic surfactants may be used, but cationic surfactants such as alkylammonium salts are preferable. As alkylammonium salts, those having a C₈ or higher alkyl group are preferable, and, in view of industrial availability, those having a C₁₂₋₁₈ alkyl group are more preferable. As specific examples of alkylammonium salts, hexadecyltrimethylammonium chloride, cetyltrimethylammonium bromide, stearyltrimethylammonium bromide, cetyltrimethylammonium chloride, stearyltrimethylammonium chloride, dodecyltrimethylammonium bromide, octadecyltrimethylammonium bromide, dodecyltrimethylammonium chloride, octadecyltrimethylammonium chloride, didodecyldimethylammonium bromide, ditetradecyldimethylammonium bromide, didodecyldimethylammonium chloride, ditetradecyldimethylammonium chloride, and the like can be mentioned. Surfactants may be used alone or as a combination of two or more kinds.

The amount of a raw material for doping copper in a mesoporous silica (in the case of using an additional metal in combination with copper, the total amount of respective raw materials) dissolved in the solvent is, per 1 mol of a silica raw material added in the below-described Step 2, for example, 0.001 to 1 mol, preferably 0.005 to 0.5 mol, and more preferably 0.01 to 0.1 mol.

As the raw material for doping copper or an other metal in addition to copper in a mesoporous silica, for example, their respective nitrate, sulfate, chloride, or oxychloride can be used. As the raw material for doping copper, it is preferable to use copper nitrate or copper chloride. As the raw material in the case of doping aluminum in addition to copper, it is preferable to use aluminum chloride. As the raw material in the case of doping zirconium, it is preferable to use zirconium oxychloride. As the raw material in the case of doping cobalt, it is preferable to use cobalt nitrate. As the raw material in the case of doping manganese, it is preferable to use manganese chloride. As the raw material in the case of doping iron, it is preferable to use iron chloride. Raw materials for doping copper or an other metal in addition to copper may be used alone or as a combination of two or more kinds.

As the solvent, for example, water can be used. The solvent may be a mixed solvent of water and a water-soluble organic solvent such as methanol, ethanol, and a polyhydric alcohol including diethylene glycol, glycerin, or the like.

### (Step 2)

Next, in the solution where the surfactant forms micelles obtained in Step 1, a silica raw material is dissolved at room temperature, for example, and stirred until uniform to allow the silica raw material to accumulate on the surface of the surfactant micelles. The amount of the silica raw material dissolved in the solution is 0.2 to 1.8 mol/L, for example. Alternatively, in the case where water or a mixed solvent of water and a water-soluble organic solvent is used as the solvent, the amount is 0.001 to 0.05 mol per 1 mol of water, for example.

The silica raw material is not particularly limited as long as it forms an inorganic network composed of mesoporous silica-forming siloxane bonds upon a dehydration condensation. As specific examples of silica raw materials, tetraalkoxysilanes, such as tetraethoxysilane, tetramethoxysilane, and tetra-n-butoxysilane, and sodium silicate can be mentioned. Tetraalkoxysilanes are preferable, and tetraethoxysilane is more preferable. Silica raw materials may be used alone or as a combination of two or more kinds.

### (Step 3)

Next, the silica raw material accumulated on the surface of the surfactant micelles is subjected to a dehydration condensation to form an inorganic network composed of mesoporous silica-forming siloxane bonds, and, at the same time, copper is incorporated through chemical bonding into the inorganic network. The dehydration condensation of the silica raw material can be caused, for example, by adding a basic aqueous solution into the system to raise the pH, followed by stirring at room temperature for 1 hour or more. The basic aqueous solution is preferably added such that the pH immediately after the addition is 8 to 14, more preferably 9 to 11. As specific examples of basic aqueous solutions, an aqueous sodium hydroxide solution, an aqueous sodium carbonate solution, and aqueous ammonia can be mentioned, and an aqueous sodium hydroxide solution is preferable. Basic aqueous solutions may be used alone or as a combination of two or more kinds. Incidentally, the dehydration condensation of the silica raw material can also be caused by adding an acidic aqueous solution such as an aqueous hydrochloric acid solution into the system to lower the pH, followed by stirring.

### (Step 4)

Finally, the surfactant micelles having formed on the surface thereof an inorganic network composed of mesoporous silica-forming siloxane bonds, into which copper has been incorporated through chemical bonding, obtained in Step 3 are collected by filtration as precipitates, dried at 30 to 70°C for 10 to 48 hours, for example, and then calcined at 400 to 600°C for 1 to 10 hours to obtain the intended copper-doped mesoporous silica. The copper-doped mesoporous silica thus obtained may be pulverized in a mixer or a mill as necessary to have the desired particle size (e. g., a median diameter of 0.01 to 100 µm).

Incidentally, the addition of the raw material for doping copper in a mesoporous silica into the system is not limited to the embodiment in which the raw material is dissolved in a solvent together with a surfactant in the above Step 1, and may also be an embodiment in which the raw material is dissolved in the solution in Step 2 or Step 3 as long as it takes place before the formation of an inorganic network composed of mesoporous silica-forming siloxane bonds upon the dehydration condensation of the silica raw material in Step 3 is completed.

In the case where the copper-doped silica is non-porous, the copper-doped non-porous silica can be produced using a precipitation process that is well known as a method for producing a non-porous silica, for example. Specifically, the production is possible by allowing a raw material for doping copper in a non-porous silica to coexist in the system during the neutralization reaction between an alkali metal silicate, which is a silica raw material, and a mineral acid. The raw material for doping copper in a non-porous silica can be the same as those for doping copper in a mesoporous silica described above. The amount of the raw material used for doping copper in a non-porous silica (in the case of using an additional metal in combination with copper, the total amount of respective raw materials) is, for example, 0.001 to 0.5 mol, preferably 0.01 to 0.1 mol, per 1 mol of an alkali metal silicate as a silica raw material. The alkali metal silicate used as a silica raw material may be a common one used for the production of a non-porous silica by a precipitation process. Specifically, sodium silicate, potassium silicate, and the like can be mentioned, and the molar ratio between silica and alkali: SiO₂/M₂O (M is Na, K, etc.) is 0.5 to 4, for example. The amount of the alkali metal silicate dissolved in a solvent (water, etc.) is, for example, 0.001 to 0.05 mol per 1 mol of a solvent. As the mineral acid, hydrochloric acid, sulfuric acid, or nitric acid can be used. The copper-doped non-porous silica is obtained as precipitates formed from the neutralization reaction between an alkali metal silicate and a mineral acid, and its collection by filtration, washing, drying, and the like can be performed in accordance with methods employed in the production of a non-porous silica by a precipitation process. As necessary, calcination at a high temperature of 300°C or more may be finally performed. The specific surface area (BET specific surface area) of the copper-doped non-porous silica thus produced is 0.1 to 350 m²/g, for example. As necessary, as in the case of the copper-doped mesoporous silica described above, the silica may be pulverized in a mixer or a mill to have the desired particle size (e.g., a median diameter of 0.01 to 100 µm).

The vinylpyrrolidone unit-containing polymer used as a resin component in the antiviral aerosol composition of the present invention may be a copolymer of a vinylpyrrolidone unit and a non-vinylpyrrolidone unit, for example. In this case, in order to facilitate the formation of a coating film whose copper-doped silica exhibits excellent antiviral properties and which is firmly retained on the surface of an article, it is preferable that the ratio between the vinylpyrrolidone unit and the non-vinylpyrrolidone unit (vinylpyrrolidone unit:non-vinylpyrrolidone unit) is 2:8 to 8:2. As specific examples of copolymers of a vinylpyrrolidone unit and a non-vinylpyrrolidone unit, a copolymer of vinylpyrrolidone and dimethylaminoethyl methacrylate, a copolymer of vinylpyrrolidone and methylvinylimidazolinium chloride, a copolymer of vinylpyrrolidone and dimethylaminopropylamide methacrylate, a copolymer of vinylpyrrolidone and quaternized imidazoline, vinylpyrrolidone, vinylcaprolactam, and methylvinylimidazolium methylsulfate, a copolymer of vinylpyrrolidone and vinyl acetate, a copolymer of vinylpyrrolidone and eicosene, a copolymer of vinylpyrrolidone and hexadecene, a copolymer of vinylpyrrolidone and styrene, and a copolymer of vinylpyrrolidone, vinylcaprolactam, and dimethylaminoethyl methacrylate can be mentioned. In addition, the vinylpyrrolidone unit-containing polymer may also be polyvinylpyrrolidone. The weight average molecular weight of the vinylpyrrolidone unit-containing polymer is, for example, 5,000 to 1,500,000, preferably 6,000 to 1,000,000, more preferably 7,000 to 500,000, and still more preferably 8,000 to 100,000. When the weight average molecular weight of the vinylpyrrolidone unit-containing polymer is too small, in the case where the copper-doped silica is porous, the molecules enter fine pores of the silica. As a result, in the case where the exhibition of characteristics due to the presence of fine pores (deodorizing properties, etc.) is expected in addition to the exhibition of antiviral properties, such exhibition may be difficult. Meanwhile, when the weight average molecular weight is too large, molecules may entirely cover the silica surface, whereby antiviral properties of the silica, which are exhibited upon virus adsorption, may be inhibited. Vinylpyrrolidone unit-containing polymers may be used alone or as a combination of two or more kinds.

In the antiviral aerosol composition of the present invention, the alcohol-based solvent may be a common one used as a component of an aerosol composition. As specific examples thereof, methanol, ethanol, and isopropyl alcohol can be mentioned. Alcohol-based solvents may be used alone or as a combination of two or more kinds. In the case where a mixed solvent of an alcohol-based solvent and water is used, the weight ratio between the alcohol-based solvent and water (alcohol-based solvent:water) is 1:5 to 100:1, for example.

In the antiviral aerosol composition of the present invention, the propellant may be a common one used as a component of an aerosol composition. As specific examples thereof, a liquefied petroleum gas, a nitrogen gas, a carbon dioxide gas, dimethyl ether, a hydrofluoroolefin, and a hydrofluorocarbon can be mentioned. Propellants may be used alone or as a combination of two or more kinds.

The contents of the copper-doped silica, the vinylpyrrolidone unit-containing polymer, the alcohol-based solvent or the mixed solvent of an alcohol-based solvent and water, and the propellant in the antiviral aerosol composition of the present invention are, for example, as follows: the content of the copper-doped silica is 0.1 to 5 wt%, the content of the vinylpyrrolidone unit-containing polymer is 0.1 to 5 wt%, the content of the alcohol-based solvent or the mixed solvent of an alcohol-based solvent and water is 30 to 90 wt%, and the content of the propellant is 5 to 65 wt%. It is preferable that the content of the copper-doped silica is 0.2 to 2 wt%, the content of the vinylpyrrolidone unit-containing polymer is 0.2 to 2 wt%, the content of the alcohol-based solvent or the mixed solvent of an alcohol-based solvent and water is 45 to 70 wt%, and the content of the propellant is 25 to 50 wt%. The antiviral aerosol composition of the present invention may incorporate pigments, fragrances, dispersants, defoamers, leveling agents, lubricants, fungicides, preservatives, waxes, and the like, commonly used as components of an aerosol composition. In the case where these components are incorporated, the content of the alcohol-based solvent or the mixed solvent of an alcohol-based solvent and water is reduced by the contents of these components. In addition, in the production of the antiviral aerosol composition of the present invention, in the case where the copper-doped silica is provided as a slurry suspended in a dispersion medium different from the alcohol-based solvent or the mixed solvent of an alcohol-based solvent and water used in the antiviral aerosol composition of the present invention, or the vinylpyrrolidone unit-containing polymer is provided as a solution dissolved in a solvent different from the alcohol-based solvent or the mixed solvent of an alcohol-based solvent and water used in the antiviral aerosol composition of the present invention, the antiviral aerosol composition of the present invention may contain such a dispersion medium or solvent. Also in this case, the content of the alcohol-based solvent or the mixed solvent of an alcohol-based solvent and water is reduced by the contents of these components.

The weight ratio between the copper-doped silica and the vinylpyrrolidone unit-containing polymer in the antiviral aerosol composition of the present invention (copper-doped silica:vinylpyrrolidone unit-containing polymer) is preferably 5:95 to 75:25, more preferably 15:85 to 70:30, and still more preferably 25:75 to 65:35. When the proportion of the copper-doped silica is too small, it may be difficult to form a coating film whose copper-doped silica exhibits excellent antiviral properties. Meanwhile, when the proportion of the vinylpyrrolidone unit-containing polymer is too small, it may be difficult to form a coating film that is firmly retained on the surface of an article.

The antiviral aerosol composition of the present invention can be produced using a common method for producing an aerosol composition in which, for example, a stock solution prepared by mixing, after adjusting the temperature as necessary, a copper-doped silica, a vinylpyrrolidone unit-containing polymer, and an alcohol-based solvent or a mixed solvent of an alcohol-based solvent and water, each to a desired content, is mixed with a propellant.

It is preferable that the mixing of a copper-doped silica, a vinylpyrrolidone unit-containing polymer, and an alcohol-based solvent or a mixed solvent of an alcohol-based solvent and water to prepare a stock solution is performed by ball-milling them. As a result of ball-milling a copper-doped silica, a vinylpyrrolidone unit-containing polymer, and an alcohol-based solvent or a mixed solvent of an alcohol-based solvent and water, a stock solution having the copper-doped silica uniformly dispersed therein without aggregation in the alcohol-based solvent or the mixed solvent of an alcohol-based solvent and water, in which the vinylpyrrolidone unit-containing polymer is uniformly dissolved, can be obtained. As result, in the formed coating film, the copper-doped silica is loaded while being uniformly dispersed without aggregation, and thus is prevented from shedding from the coating film. At the same time, despite the coexistence of the copper-doped silica, the vinylpyrrolidone unit-containing polymer acquires high adhesion to the surface of an article, making it easy to form a coating film whose copper-doped silica exhibits excellent antiviral properties and which is firmly retained on the surface of an article. In addition, this makes it easy to increase the stability of the antiviral aerosol composition of the present invention and perform aerosol application without causing nozzle clogging or the like.

Ball milling can be performed by housing a copper-doped silica, a vinylpyrrolidone unit-containing polymer, and an alcohol-based solvent or a mixed solvent of an alcohol-based solvent and water in a treatment container together with balls used for ball milling (media), then putting the treatment container housing them on a ball mill stand, and rotating the same (the rotation speed employed is within a range of 15 to 2,000 rpm, for example). The time of ball milling is, for example, 1 to 50 hours, preferably 6 to 30 hours. With respect to balls used for ball milling (e.g., 1 to 5 mmϕ alumina balls or zirconia balls), it is preferable to use the number of balls weighing 1 to 5 times the total weight of the copper-doped silica, the vinylpyrrolidone unit-containing polymer, the alcohol-based solvent or the mixed solvent of an alcohol-based solvent and water, other components incorporated as necessary, the dispersion medium used in the case where the copper-doped silica is provided as a slurry suspended in a dispersion medium different from the alcohol-based solvent or the mixed solvent of an alcohol-based solvent and water used in the antiviral aerosol composition of the present invention, and the solvent used in the case where the vinylpyrrolidone unit-containing polymer is provided as a solution dissolved in a solvent different from the alcohol-based solvent or the mixed solvent of an alcohol-based solvent and water used in the antiviral aerosol composition of the present invention. The alcohol-based solvent or the mixed solvent of an alcohol-based solvent and water to be housed in the treatment container may be part of the content in the antiviral aerosol composition of the present invention. In this case, after ball milling, an alcohol-based solvent or a mixed solvent of an alcohol-based solvent and water may be added to achieve the content in the antiviral aerosol composition of the present invention.

The antiviral aerosol composition of the present invention can be unitized for imparting antiviral properties to articles around us, such as textile products, nonwoven fabric products, leather products, building materials, wood, glass, plastics, films, ceramics, paper, pulp, stationeries, toys, containers, caps, pouring tools, and spouts. The application of the antiviral aerosol composition of the present invention to the surface of an article can be performed such that, for example, the antiviral aerosol composition of the present invention is filled into a spray can and aerosol-applied to the surface of an article. The application of the antiviral aerosol composition of the present invention to the surface of an article is followed by natural drying or heat drying, whereby a coating film whose copper-doped silica exhibits excellent antiviral properties and which is firmly retained on the surface of an article can be formed.

In the coating film formed from the antiviral aerosol composition of the present invention, the copper-doped silica contained in the coating film exhibits excellent antiviral action not only against enveloped viruses but also against non-enveloped viruses. Therefore, the kinds of viruses that are targeted by the antiviral action are not limited and include various pathogenic viruses. For example, as enveloped viruses, influenza A, B, and C viruses, parainfluenza virus, mumps virus, measles virus, human metapneumovirus, RS virus, Nipah virus, Hendra virus, yellow fever virus, dengue virus, Japanese encephalitis virus, West Nile virus, hepatitis B, C, and D viruses, eastern and western equine encephalitis viruses, rubella virus, Lassa virus, Junin virus, Machupo virus, Guanarito virus, Sabia virus, Crimean-Congo hemorrhagic fever virus, sandfly fever virus, Hantavirus, Sin Nombre virus, Rabies virus, Ebola virus, Marburg virus, bat lyssavirus, human T-cell leukemia virus, human immunodeficiency virus, human coronavirus, SARS coronavirus, SARS-CoV-2 virus (COVID-19 virus), herpes virus, varicella zoster virus, EB virus, cytomegalovirus, smallpox virus, monkeypox virus, cowpox virus, Molluscipoxvirus, Parapoxvirus, O'nyong-nyong virus, and the like can be targeted, and, as non-enveloped viruses, rhinovirus, poliovirus, foot-and-mouth disease virus, rotavirus, norovirus, enterovirus, hepatovirus, astrovirus, sapovirus, hepatitis A and E viruses, human parvovirus, polyomavirus, human papillomavirus, adenovirus, coxsackievirus, and the like can be targeted.

In addition, the copper-doped silica contained in the antiviral aerosol composition of the present invention exhibits excellent antiviral action even at the time from the application of the antiviral aerosol composition of the present invention to the surface of an article to the formation of a coating film. At this time, the alcohol-based solvent or the mixed solvent of an alcohol-based solvent and water contained in the antiviral aerosol composition of the present invention also exhibits antiviral action.

### Examples

Hereinafter, the present invention will be described in detail with reference to examples. However, the present invention should not be construed as being limited to the following descriptions.

### Production Reference Example 1: Production of Copper- and Aluminum-Doped Mesoporous Silica

Hexadecyltrimethylammonium chloride as a surfactant, copper chloride as a raw material for doping copper in a mesoporous silica, and aluminum chloride as a raw material for doping aluminum in a mesoporous silica were dissolved in water as a solvent, stirred at 100°C for 1 hour, and then cooled to room temperature. After that, tetraethoxysilane as a silica raw material was further dissolved and stirred until uniform. Next, an aqueous sodium hydroxide solution as a basic aqueous solution was added to the reaction liquid such that the pH immediately after the addition was 9, and stirred at room temperature for 20 hours. The formed precipitates were collected by filtration, dried at 50°C for 24 hours, and then calcined at 570°C for 5 hours, thereby giving the intended copper- and aluminum-doped mesoporous silica as a slightly bluish white powder.

Incidentally, the amounts of hexadecyltrimethylammonium chloride as a surfactant, copper chloride as a raw material for doping copper in a mesoporous silica, aluminum chloride as a raw material for doping aluminum in a mesoporous silica, and water as a solvent used per 1 mol of tetraethoxysilane as a silica raw material were each as follows.
Hexadecyltrimethylammonium chloride: 0.225 mol
Copper chloride: 0.0204 mol
Aluminum chloride: 0.0482 mol
Water: 125 mol

In addition, for the preparation of the aqueous sodium hydroxide solution as a basic aqueous solution, 0.195 mol of sodium hydroxide was used per 1 mol of tetraethoxysilane as a silica raw material.

The copper- and aluminum-doped mesoporous silica obtained by the above method had a specific surface area of 1100 m²/g, and the fine pore diameter was about 2.5 nm (calculated by BJH calculation from the adsorption isotherm of nitrogen gas measured at liquid nitrogen temperature by the multi-point method using BELSORP MAX II manufactured by MicrotracBEL Corp.). In addition, about 50 mg of the copper- and aluminum-doped mesoporous silica was accurately weighed out and dissolved in 4 mL of hydrochloric acid, and then the concentrations of copper and aluminum in the hydrochloric acid solution were measured using an Inductively Coupled Plasma-Optical Emission Spectrometer (iCAP PRO XP ICP-OES manufactured by Thermo Fisher Scientific, same hereinafter). Based on the measurement results, the copper and aluminum contents in the copper- and aluminum-doped mesoporous silica were calculated. As a result, the copper content was 2.09 wt%, and the aluminum content was 2.00 wt%. The doping of the mesoporous silica with copper and aluminum was confirmed using an X-ray photoelectron spectrometer (K-Alpha Surface Analysis manufactured by Thermo Fisher Scientific, same hereinafter) and a transmission electron microscope (JEM2010 manufactured by JEOL Ltd., same hereinafter).

### Production Example 1: Production of Antiviral Aerosol Composition of the Present Invention (No. 1)

### (Step 1)

In a 2 L I-Boy PP wide-mouth bottle, 140 g of the copper- and aluminum-doped mesoporous silica obtained in Production Reference Example 1, 560 g of ethanol, and 1760 g of 5 mmϕ alumina balls were placed. At room temperature, the bottle was put on a ball mill stand and treated at a rotation speed of 250 rpm for 1 hour and further at 450 rpm for 9 hours, and then the alumina balls were removed, thereby giving an ethanol slurry in which the content of the copper- and aluminum-doped mesoporous silica having a median diameter of about 0.6 µm (the median diameter was measured using a laser diffraction particle size distribution analyzer (SALD-3100 manufactured by Shimadzu Corporation), same hereinafter) was 20 wt%.

### (Step 2)

In a 2 L I-Boy PP wide-mouth bottle, 35 g of a powder of a copolymer of vinylpyrrolidone and vinyl acetate (PVP/VA S630, manufactured by Ashland Inc., ratio between vinylpyrrolidone and vinyl acetate units = 6:4, weight average molecular weight (catalog value): 51,000, same hereinafter) and 490 g of ethanol were placed to dissolve the powder of the copolymer of vinylpyrrolidone and vinyl acetate in ethanol, and then 175 g of the ethanol slurry in which the content of the copper- and aluminum-doped mesoporous silica having a median diameter of about 0.6 µm was 20 wt% obtained in Step 1 and 1760 g of 2 mmϕ alumina balls were placed. At room temperature, the bottle was put on a ball mill stand and treated at a rotation speed of 500 rpm for 9 hours, and then the alumina balls were removed, thereby giving a coating composition in which the content of the copper- and aluminum-doped mesoporous silica having a median diameter of about 0.5 µm was 5 wt%, the content of the copolymer of vinylpyrrolidone and vinyl acetate was 5 wt%, and the content of ethanol was 90 wt%. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%.

### (Step 3)

The stock solution obtained in Step 2 and a liquefied petroleum gas as a propellant (vapor pressure at 20°C: 0.29 MPa, same hereinafter) were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.6 wt%, the content of ethanol was 58.8 wt%, and the content of the liquefied petroleum gas was 40.0 wt%.

### Production Example 2: Production of Antiviral Aerosol Composition of the Present Invention (No. 2)

In a 2 L I-Boy PP wide-mouth bottle, 35 g of the copper- and aluminum-doped mesoporous silica obtained in Production Reference Example 1, 315 g of ethanol, 350 g of a 10 wt% ethanol solution of a powder of a copolymer of vinylpyrrolidone and vinyl acetate (PVP/VA S630), and 1700 g of 2 mmϕ alumina balls were placed. At room temperature, the bottle was put on a ball mill stand and treated at a rotation speed of 900 rpm for 24 hours, and then the alumina balls were removed, thereby giving a coating composition in which the content of the copper- and aluminum-doped mesoporous silica having a median diameter of about 0.5 µm was 5 wt%, the content of the copolymer of vinylpyrrolidone and vinyl acetate was 5 wt%, and the content of ethanol was 90 wt%. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.6 wt%, the content of ethanol was 58.8 wt%, and the content of the liquefied petroleum gas was 40.0 wt%.

### Production Example 3: Production of Antiviral Aerosol Composition of the Present Invention (No. 3)

### (Step 1)

In a 250 mL I-Boy PP wide-mouth bottle, 4.4 g of the copper- and aluminum-doped mesoporous silica obtained in Production Reference Example 1, 83.6 g of ethanol, and 220 g of 2 mmϕ alumina balls were placed. At room temperature, the bottle was put on a ball mill stand and treated at a rotation speed of 180 rpm for 8 hours, and then the alumina balls were removed, thereby giving an ethanol slurry in which the content of the copper- and aluminum-doped mesoporous silica having a median diameter of about 0.5 µm was 5 wt%.

### (Step 2)

The ethanol slurry in which the content of the copper- and aluminum-doped mesoporous silica having a median diameter of about 0.5 µm was 5 wt% obtained in Step 1, a powder of a copolymer of vinylpyrrolidone and vinyl acetate (PVP/VA S630), and ethanol were mixed in a mass ratio of 20:1:79 and stirred, thereby giving, as a stock solution, a coating composition in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%.

### (Step 3)

The stock solution obtained in Step 2 and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.6 wt%, the content of ethanol was 58.8 wt%, and the content of the liquefied petroleum gas was 40.0 wt%.

### Production Example 4: Production of Antiviral Aerosol Composition of the Present Invention (No. 4)

In the same manner as in Production Example 3 except that the mixing of the ethanol slurry in which the content of the copper- and aluminum-doped mesoporous silica having a median diameter of about 0.5 µm was 5 wt% obtained in Step 1, a powder of a copolymer of vinylpyrrolidone and vinyl acetate (PVP/VA S630), and ethanol in Step 2 of Production Example 3 was performed in a mass ratio of 40:1:159, a coating composition, in which the content of the copper- and aluminum-doped mesoporous silica having a median diameter of about 0.5 µm was 1 wt%, the content of the copolymer of vinylpyrrolidone and vinyl acetate was 0.5 wt%, and the content of ethanol was 98.5 wt%, was obtained as a stock solution. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.3 wt%, the content of ethanol was 59.1 wt%, and the content of the liquefied petroleum gas was 40.0 wt%.

### Production Example 5: Production of Antiviral Aerosol Composition of the Present Invention (No. 5)

In a 2 L I-Boy PP wide-mouth bottle, 70 g of a 50 wt % aqueous solution of a copolymer of vinylpyrrolidone and vinyl acetate (PVP/VA W735, manufactured by Ashland Inc., ratio between vinylpyrrolidone and vinyl acetate units = 7:3, weight average molecular weight (catalog value): 27,300), 455 g of ethanol, 175 g of the ethanol slurry in which the content of the copper- and aluminum-doped mesoporous silica having a median diameter of about 0.6 µm was 20 wt% obtained in Step 1 of Production Example 1, and 1760 g of 2 mmϕ alumina balls were placed. At room temperature, the bottle was put on a ball mill stand and treated at a rotation speed of 500 rpm for 9 hours, and then the alumina balls were removed, thereby giving a coating composition in which the content of the copper- and aluminum-doped mesoporous silica having a median diameter of about 0.5 µm was 5 wt%, the content of the copolymer of vinylpyrrolidone and vinyl acetate was 5 wt%, the content of water was 5 wt%, and the content of ethanol was 85 wt%. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, the content of water was 1 wt%, and the content of ethanol was 97 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.6 wt%, the content of water was 0.6 wt%, the content of ethanol was 58.2 wt%, and the content of the liquefied petroleum gas was 40.0 wt%.

### Production Example 6: Production of Antiviral Aerosol Composition of the Present Invention (No. 6)

In the same manner as in Production Example 5 except that a 50 wt% ethanol solution of a copolymer of vinylpyrrolidone and vinyl acetate (PVP/VA E535, manufactured by Ashland Inc., ratio between vinylpyrrolidone and vinyl acetate units = 5:5, weight average molecular weight (catalog value): 36,700) was used in place of the 50 wt% aqueous solution of the copolymer of vinylpyrrolidone and vinyl acetate (PVP/VA W735), a coating composition, in which the content of the copper- and aluminum-doped mesoporous silica having a median diameter of about 0.5 µm was 5 wt%, the content of the copolymer of vinylpyrrolidone and vinyl acetate was 5 wt%, and the content of ethanol was 90 wt%, was obtained. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.6 wt%, the content of ethanol was 58.8 wt%, and the content of the liquefied petroleum gas was 40.0 wt%.

### Production Example 7: Production of Antiviral Aerosol Composition of the Present Invention (No. 7)

In the same manner as in Production Example 5 except that a 50 wt% ethanol solution of a copolymer of vinylpyrrolidone and vinyl acetate (PVP/VA E335, manufactured by Ashland Inc., ratio between vinylpyrrolidone and vinyl acetate units = 3:7, weight average molecular weight (catalog value): 28,800) was used in place of the 50 wt% aqueous solution of the copolymer of vinylpyrrolidone and vinyl acetate (PVP/VA W735), a coating composition, in which the content of the copper- and aluminum-doped mesoporous silica having a median diameter of about 0.5 µm was 5 wt%, the content of the copolymer of vinylpyrrolidone and vinyl acetate was 5 wt%, and the content of ethanol was 90 wt%, was obtained. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of the copolymer of vinylpyrrolidone and vinyl acetate was 0.6 wt%, the content of ethanol was 58.8 wt%, and the content of the liquefied petroleum gas was 40.0 wt%.

### Production Example 8: Production of Antiviral Aerosol Composition of the Present Invention (No. 8)

In the same manner as in Production Example 1 except that a powder of polyvinylpyrrolidone (PVP K15, manufactured by Ashland Inc., weight average molecular weight (catalog value): 8,000) was used in place of the powder of the copolymer of vinylpyrrolidone and vinyl acetate (PVP/VA S630), a coating composition, in which the content of the copper- and aluminum-doped mesoporous silica having a median diameter of about 0.5 µm was 5 wt%, the content of polyvinylpyrrolidone was 5 wt%, and the content of ethanol was 90 wt%, was obtained. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of polyvinylpyrrolidone was 0.6 wt%, the content of ethanol was 58.8 wt%, and the content of the liquefied petroleum gas was 40.0 wt%.

### Production Example 9: Production of Antiviral Aerosol Composition of the Present Invention (No. 9)

In the same manner as in Production Example 1 except that a powder of polyvinylpyrrolidone (PVP K30, manufactured by Ashland Inc., weight average molecular weight (catalog value): 60,000) was used in place of the powder of the copolymer of vinylpyrrolidone and vinyl acetate (PVP/VA S630), a coating composition, in which the content of the copper- and aluminum-doped mesoporous silica having a median diameter of about 0.5 µm was 5 wt%, the content of polyvinylpyrrolidone was 5 wt%, and the content of ethanol was 90 wt%, was obtained. The obtained coating composition and ethanol were mixed in a weight ratio of 1:4, thereby giving a stock solution in which the content of the copper- and aluminum-doped mesoporous silica having a median diameter of about 0.5 µm was 1 wt%, the content of the copolymer of vinylpyrrolidone and vinyl acetate was 1 wt%, and the content of ethanol was 98 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped mesoporous silica having a median diameter of about 0.5 µm was 0.6 wt%, the content of polyvinylpyrrolidone was 0.6 wt%, the content of ethanol was 58.8 wt%, and the content of the liquefied petroleum gas was 40.0 wt%.

### Test Example 1: Antiviral Action against Enveloped Virus of Coating Film Formed from Antiviral Aerosol Composition of the Present Invention

### (1) Formation of Coating Film through Aerosol Application

An aerosol can filled with the antiviral aerosol composition of the present invention produced in Production Example 1 was held horizontally, and, from a height of 15 cm, the composition was sprayed uniformly over the entire surface of a 10 cm × 20 cm PET film for 7 seconds (spray amount: 1.36 g/sec) and dried naturally, thereby forming a coating film on the surface of the PET film. During the spraying of the antiviral aerosol composition of the present invention produced in Production Example 1 from the aerosol can, problems with spraying due to nozzle clogging or the like were not seen. The coating film formed on the surface of the PET film was white, and the loading amount of the copper- and aluminum-doped mesoporous silica having a median diameter of about 0.5 µm was 0.27 mg/cm². Incidentally, the loading amount of the mesoporous silica was calculated as follows. The PET film having a white coating film formed on its surface was placed in a crucible, 6 mol/L hydrochloric acid was added, followed by heating at 120°C for 1 hour, and then the amount of copper in the crucible was measured using an Inductively Coupled Plasma-Optical Emission Spectrometer; the calculation was performed based on the amount of copper thus measured.

### (2) Evaluation of Antiviral Action

MDCK cells (cells derived from canine kidney) as host cells were infected with influenza A virus (H3N2, A/Hong Kong/8/68; TC adapted, ATCC VR-1679) and cultured. Subsequently, the cell debris was removed by centrifugation to give a virus suspension (infectivity titer: 4.7 × 10⁷ PFU/mL). In accordance with ISO 21702, 0.4 mL of this virus suspension was dropped on a 5 cm square test piece cut out from the PET film having a white coating film formed on its surface, covered with a 4 cm square cover film, and then left at 25°C. After 24 hours, a virus washout solution was added to the test piece to wash out the virus, and the viral infectivity titer was measured by the plaque assay method. The result is shown in Table 1. Incidentally, Table 1 also shows the results of the same tests performed using the antiviral aerosol compositions of the present invention produced in Production Examples 5 to 7, as well as the result of the case where, as a control, the virus suspension was dropped on a 5 cm square PET film.

**[Table 1]**

| Specimen | Aerosol Composition | Resin Component | | | Viral Infectivity Titer (PFU/cm²) Average Common Logarithm | | Antiviral Activity Value [*R*] |
|---|---|---|---|---|---|---|---|
| | | Kind | VP:VA | Mw | Immediately after inoculation | After 24 hours | |
| Coating Film-Formed PET Film | Production Example 1 | PVP/VA | 6:4 | 51,000 | Not measured | 0.90 [*A*ₜ] | 4.4 |
| Coating Film-Formed PET Film | Production Example 5 | PVP/VA | 7:3 | 27,300 | Not measured | < 0.80 [*A*ₜ] | ≥ 4.5 |
| Coating Film-Formed PET Film | Production Example 6 | PVP/VA | 5:5 | 36,700 | Not measured | < 0.80 [*A*ₜ] | ≥ 4.5 |
| Coating Film-Formed PET Film | Production Example 7 | PVP/VA | 3:7 | 28,800 | Not measured | < 0.80 [*A*ₜ] | ≥ 4.5 |
| Untreated PET Film | - | - | - | - | 6.07 | 5.30 [*U*ₜ] | - |

| | | | | | | | |
|---|---|---|---|---|---|---|---|
| PVP/VA : Copolymer of vinylpyrrolidone and vinyl acetate VP:VA: Ratio between vinylpyrrolidone and vinyl acetate units Mw: Weight average molecular weight (catalog value) Antiviral activity value [*R*] = [*U*ₜ] - [*A*ₜ] | | | | | | | |

As is clear from Table 1, the coating films formed from the antiviral aerosol compositions of the present invention produced in Production Examples 1 and 5 to 7 exhibited excellent antiviral action against influenza virus, which is an enveloped virus.

### Test Example 2: Antiviral Action against Non-Enveloped Virus of Coating Film Formed from Antiviral Aerosol Composition of the Present Invention

### (1) Formation of Coating Film through Aerosol Application

From the antiviral aerosol composition of the present invention produced in Production Example 1, a white coating film was formed on the surface of a 10 cm × 20 cm PET film in the same manner as in Test Example 1.

### (2) Evaluation of Antiviral Action

CRFK cells (cells derived from feline kidney) as host cells were infected with feline calicivirus (F-9, Feline calicivirus; Strain: F-9, ATCC VR-782) as a surrogate virus for norovirus, and cultured. Subsequently, the cell debris was removed by centrifugation to give a virus suspension (infectivity titer: 2.4 × 10⁷ PFU/mL). In accordance with ISO 21702, 0.4 mL of this virus suspension was dropped on a 5 cm square test piece cut out from the PET film having a white coating film formed on its surface, covered with a 4 cm square cover film, and then left at 25°C. After 24 hours, a virus washout solution was added to the test piece to wash out the virus, and the viral infectivity titer was measured by the plaque assay method. The result is shown in Table 2. Incidentally, Table 2 also shows the results of the same tests performed using the antiviral aerosol compositions of the present invention produced in Production Examples 5 to 7, as well as the result of the case where, as a control, the virus suspension was dropped on a 5 cm square PET film.

**[Table 2]**

| Specimen | Aerosol Composition | Resin Component | | | Viral Infectivity Titer (PFU/cm²) Average Common Logarithm | | Antiviral Activity Value [*R*] |
|---|---|---|---|---|---|---|---|
| | | Kind | VP:VA | Mw | Immediately after inoculation | After 24 hours | |
| Coating Film-Formed PET Film | Production Example 1 | PVP/VA | 6:4 | 51,000 | Not measured | 0.90 [*A*ₜ] | 4.5 |
| Coating Film-Formed PET Film | Production Example 5 | PVP/VA | 7:3 | 27,300 | Not measured | 1.10 [*A*ₜ] | 4.3 |
| Coating Film-Formed PET Film | Production Example 6 | PVP/VA | 5:5 | 36,700 | Not measured | 0.80 [*A*ₜ] | 4.6 |
| Coating Film-Formed PET Film | Production Example 7 | PVP/VA | 3:7 | 28,800 | Not measured | < 0.80 [*A*ₜ] | ≥ 4.6 |
| Untreated PET Film | - | - | - | - | 5.70 | 5.42 [*U*ₜ] | - |

| | | | | | | | |
|---|---|---|---|---|---|---|---|
| PVP/VA: Copolymer of vinylpyrrolidone and vinyl acetate VP:VA: Ratio between vinylpyrrolidone and vinyl acetate units Mw: Weight average molecular weight (catalog value) Antiviral activity value [*R*] = [*U*ₜ] - [*A*ₜ] | | | | | | | |

As is clear from Table 2, the coating films formed from the antiviral aerosol compositions of the present invention produced in Production Examples 1 and 5 to 7 exhibited excellent antiviral action also against feline calicivirus, which is a non-enveloped virus.

### Test Example 3: Adsorption Action for Hemagglutinin (HA) of Coating Film Formed from Antiviral Aerosol Composition of the Present Invention

The adsorption action of a coating film formed from the antiviral aerosol composition of the present invention for HA, the surface protein of influenza virus, was evaluated using HA (H1N1) (A/California/06/2009 Hemagglutinin ELISA Development Kit (Immune Technology Corp., IT-E3Ag-2009H1N1-California/06). Specifically, from a 10 cm × 20 cm PET film having on its surface a white coating film formed from the antiviral aerosol composition of the present invention produced in Production Example 1 in the same manner as in Test Example 1, a test piece having an area of 7.4 cm² (the loading amount of the copper- and aluminum-doped mesoporous silica having a median diameter of about 0.5 µm: 2 mg) was cut out, immersed in 2 mL of an HA solution having an initial concentration of 25.3 ng/mL, stirred for 20 seconds using a vortex mixer, and allowed to stand at room temperature. After 3 minutes, the film was taken out, the solution was centrifuged to settle the solids, the supernatant was collected and measured for its HA concentration, and the adsorption rate for HA was calculated from the mathematical formula: Adsorption rate (%) =

((Initial HA concentration - HA concentration after 3 minutes) /Initial HA concentration) × 100. Table 3 shows the relative value of the calculated adsorption rate for HA, taking, as 100, the adsorption rate of a copper- and aluminum-doped mesoporous silica having a median diameter of about 0.5 µm for HA calculated in the same manner using 2 mL of an HA solution having an initial concentration of 25.3 ng/mL having added thereto 2 mg of the mesoporous silica. Incidentally, Table 3 also shows the results of the same tests performed using the antiviral aerosol compositions of the present invention produced in Production Examples 8 and 9.

**[Table 3]**

| Specimen | Aerosol Composition | Silica Content | Resin Component | | | Adsorption Rate (Relative Value) |
|---|---|---|---|---|---|---|
| | | | Kind | VP:VA | Mw | |
| Cu,Al-Doped Mesoporous Silica | | 0.1 wt% | - | - | - | 100 |
| Coating Film-Formed PET Film | Production Example 1 | 0.1 wt% | PVP/VA | 6:4 | 51,000 | 94.7 |
| Coating Film-Formed PET Film | Production Example 8 | 0.1 wt% | PVP | | 8,000 | 94.9 |
| Coating Film-Formed PET Film | Production Example 9 | 0.1 wt% | PVP | | 60,000 | 86.8 |

| | | | | | | |
|---|---|---|---|---|---|---|
| PVP/VA: Copolymer of vinylpyrrolidone and vinyl acetate VP:VA: Ratio between vinylpyrrolidone and vinyl acetate units PVP: Polyvinylpyrrolidone Mw: Weight average molecular weight (catalog value) | | | | | | |

As is clear from Table 3, the adsorption rate for HA of the coating film formed from the antiviral aerosol composition of the present invention produced in Production Example 1 was almost equal to the adsorption rate for HA of the copper- and aluminum-doped mesoporous silica having a median diameter of about 0.5 um, and almost no decrease in the adsorption rate for HA of the mesoporous silica due to the coexistence with the copolymer of vinylpyrrolidone and vinyl acetate in the coating film was seen. From this, the excellent adsorption action for HA of the coating film formed from the antiviral aerosol composition of the present invention produced in Production Example 1 was confirmed. Although the excellent adsorption action for HA of the coating films formed from the antiviral aerosol compositions of the present invention produced in Production Examples 8 and 9 was also confirmed, it was presumed that there was a decreasing trend of the action with an increase in the weight average molecular weight of polyvinylpyrrolidone.

### Test Example 4: Durability of Coating Film Formed from Antiviral Aerosol Composition of the Present Invention

An aerosol can filled with the antiviral aerosol composition of the present invention produced in Production Example 1 was held horizontally, and, from a height of 15 cm, the composition was sprayed onto a PET film for 1 second and dried naturally, thereby giving a PET film having a white coating film on its surface with a diameter of about 6 cm. From the PET film, two test pieces (test piece A and test piece B) each measuring 2 cm × 4 cm were cut out. Sellotape (registered trademark, same hereinafter) was tightly attached to the entire surface of the coating film of the test piece A by pressing with hand, and then immediately peeled off. The test piece A after removing the Sellotape and the test piece B were each placed in a crucible, 6 mol/L hydrochloric acid was added, followed by heating at 120°C for 1 hour, and then the amount of copper in the crucible was measured using an Inductively Coupled Plasma-Optical Emission Spectrometer to determine the amount of copper in each of the test piece A after removing the Sellotape and the test piece B. The durability of the coating film was evaluated from the mathematical formula: Coating film residual rate (%) = (Amount of copper in test piece A after removing Sellotape/Amount of copper in test piece B) × 100. The result is shown in Table 4. Incidentally, Table 4 also shows the results of the same tests performed using the antiviral aerosol compositions of the present invention produced in Production Examples 4 and 5, as well as the result of the same test performed in which, as a control, a stock solution in which the content of the copper- and aluminum-doped mesoporous silica having a median diameter of about 0.5 µm was 1 wt% and the content of ethanol was 99 wt%, and a liquefied petroleum gas as a propellant were filled into an aerosol can in a weight ratio of 6:4.

**[Table 4]**

| Specimen | Aerosol Composition | Stock Solution | | | | | | | Coating Film Residual Rate |
|---|---|---|---|---|---|---|---|---|---|
| | | Silica Content | Resin Component | | | | Solvent Content | | |
| | | | Kind | VP:VA | Mw | Content | Ethanol | Water | |
| Coating Film-Formed PET Film | Production Example 1 | 1 wt% | PVP/VA | 6:4 | 51,000 | 1 wt% | 98 wt% | | 68% |
| Coating Film-Formed PET Film | Production Example 4 | 1 wt% | PVP/VA | 6:4 | 51,000 | 0.5 wt% | 98.5 wt% | | 56% |
| Coating Film-Formed PET Film | Production Example 5 | 1 wt% | PVP/VA | 7:3 | 27,300 | 1 wt% | 97 wt% | 1 wt% | 80% |
| Coating Film-Formed PET Film | - | 1 wt% | - | - | - | - | 99 wt% | | 8% |

| | | | | | | | | | |
|---|---|---|---|---|---|---|---|---|---|
| PVP/VA: Copolymer of vinylpyrrolidone and vinyl acetate VP:VA: Ratio between vinylpyrrolidone and vinyl acetate units Mw: Weight average molecular weight (catalog value) | | | | | | | | | |

As is clear from Table 4, as a result of using a vinylpyrrolidone unit-containing polymer as a resin component of an aerosol composition, it was possible to form a coating film firmly retained on the surface of a PET film by aerosol application.

### Test Example 5: Spray Deposition Properties of Antiviral Aerosol Composition of the Present Invention

When an aerosol composition is sprayed onto the surface of a target object, due to strong air currents, the stock solution is partially stirred up from the surface of the target object and splashed without being deposited. Because of such a phenomenon, the spray deposition properties of an aerosol composition can be evaluated from the mathematical formula: Spray deposition rate (%) =

(Actual amount of stock solution applied per unit area/Theoretical amount of stock solution applied per unit area) × 100. Here, the actual amount of the stock solution applied per unit area is calculated by dividing the amount of a deposited material on the surface of the target object immediately after spraying the aerosol composition by the application area, while the theoretical amount of the stock solution applied per unit area is calculated by dividing the theoretical amount of the stock solution applied by the application area. The theoretical amount of the stock solution applied is calculated from the mathematical formula: (Amount of aerosol composition before spraying - Amount of aerosol composition after spraying) × Proportion of stock solution in aerosol composition. The spray deposition properties of the antiviral aerosol composition of the present invention produced in Production Example 1 in the formation of a coating film in Test Example 4 was evaluated according to this method. The result is shown in Table 5. Incidentally, Table 5 also shows the results of the same tests performed using the antiviral aerosol compositions of the present invention produced in Production Examples 4 and 5, as well as the result of the same test performed in which, as a control, a stock solution in which the content of the copper- and aluminum-doped mesoporous silica having a median diameter of about 0.5 µm was 1 wt% and the content of ethanol was 99 wt%, and a liquefied petroleum gas as a propellant were filled into an aerosol can in a weight ratio of 6:4.

**[Table 5]**

| Specimen | Aerosol Composition | Stock Solution | | | | | | | Spray Deposition Rate |
|---|---|---|---|---|---|---|---|---|---|
| | | Silica Content | Resin Component | | | | Solvent Content | | |
| | | | Kind | VP:VA | Mw | Content | Ethanol | Water | |
| Coating Film-Formed PET Film | Production Example 1 | 1 wt% | PVP/VA | 6:4 | 51,000 | 1 wt% | 98 wt% | | 71% |
| Coating Film-Formed PET Film | Production Example 4 | 1 wt% | PVP/VA | 6:4 | 51,000 | 0.5 wt% | 98.5 wt% | | 45% |
| Coating Film-Formed PET Film | Production Example 5 | 1 wt% | PVP/VA | 7:3 | 27,300 | 1 wt% | 97 wt% | 1 wt% | 33% |
| Coating Film-Formed PET Film | - | 1 wt% | - | - | - | - | 99 wt% | | 20% |

| | | | | | | | | | |
|---|---|---|---|---|---|---|---|---|---|
| PVP/VA: Copolymer of vinylpyrrolidone and vinyl acetate VP:VA: Ratio between vinylpyrrolidone and vinyl acetate units Mw: Weight average molecular weight (catalog value) | | | | | | | | | |

As is clear from Table 5, as a result of using a vinylpyrrolidone unit-containing polymer as a resin component of an aerosol composition, it was possible to increase the spray deposition rate of the aerosol composition. Considering the results of Test Example 5 together with the results of Test Example 4, it turned out that the antiviral aerosol composition of the present invention was capable of forming a coating film firmly retained on the surface of a PET film by aerosol application at a high spray deposition rate.

### Test Example 6: Stability of Antiviral Aerosol Composition of the Present Invention

20 g of each of the antiviral aerosol compositions of the present invention produced in Production Examples 1 to 9 was filled into a 100 mL pressure-resistant glass bottle, and the appearance was observed to examine the following five items. As a result, for all the items, none of the antiviral aerosol compositions of the present invention showed a practically problematic phenomenon, and it turned out that they had high stability.
- Whether the stock solution components, excluding the mesoporous silica, are compatible with the liquefied petroleum gas
- Whether deposits are formed
- Whether the composition is discolored due to the elution of copper or aluminum
- Whether the precipitated mesoporous silica can be redispersed by shaking
- Whether there is aggregation of the mesoporous silica

Even when changes were made to the composition of the antiviral aerosol composition of the present invention produced in Production Example 1, that is, the content of the mesoporous silica was increased to 3 wt% (the content of ethanol was reduced by the increment), the content of the copolymer of vinylpyrrolidone and vinyl acetate was increased to 3 wt% (the content of ethanol was reduced by the increment), the content of ethanol was reduced to 40 wt% (the content of the liquefied petroleum gas was increased by the decrement), the content of ethanol was increased to 90 wt% (the content of the liquefied petroleum gas was reduced by the increment), the content of the liquefied petroleum gas was reduced to 10 wt% (the content of ethanol was increased by the decrement), the content of the liquefied petroleum gas was increased to 60 wt% (the content of ethanol was reduced by the increment), the liquefied petroleum gas was changed to a hydrofluoroolefin (e.g., HFO-1234ze), or the liquefied petroleum gas was mixed with a nitrogen gas or a carbon dioxide gas, there was not much difference in the evaluation of stability. In addition, even when ethanol (58.8 wt%) was changed to ethanol (10.8 wt%) + water (48.0 wt%), and the liquefied petroleum gas was changed to dimethyl ether, there was not much difference in the evaluation of stability. Even when the antiviral aerosol composition of the present invention produced in Production Example 9 was subjected to the same compositional changes as those made to the antiviral aerosol composition of the present invention produced in Production Example 1, there was not much difference in the evaluation of stability.

However, when the copolymer of vinylpyrrolidone and vinyl acetate in the antiviral aerosol composition of the present invention produced in Production Example 1 was changed to any one of ethyl cellulose (Antaron ECo ethylcellulose, manufactured by Ashland Inc.), an (octyl acrylamide/hydroxypropyl acrylate/butylaminoethyl methacrylate) copolymer (AMPHOMER, manufactured by Nouryon), diisostearoyl polyglyceryl-3 dimer dilinoleate, caprylic/capric triglyceride (SolAmaze, manufactured by Nouryon), and polyquaternium-51 (LIPIDURE-PMB, manufactured by NOF Corporation), which are known as resin components of aerosol compositions, and the resulting aerosol compositions were examined for the above five items, all the aerosol compositions showed a practically problematic phenomenon for at least one item, and it turned out that they were inferior in stability.

### Production Reference Example 2: Production of Copper- and Aluminum-Doped Non-Porous Silica

A solution (a) of sodium silicate as a silica raw material (sodium metasilicate (9-hydrate): manufactured by Nippon Chemical Industrial Co., Ltd., SiO₂/Na₂O = 0.9 to 1.1 (manufacturer value)) dissolved in water as a solvent and a solution (b) of copper chloride as a raw material for doping copper in a non-porous silica and aluminum chloride as a raw material for doping aluminum in a non-porous silica dissolved in water as a solvent were each prepared. The solution (a) and the solution (b) were mixed at room temperature and stirred for 5 minutes, and then hydrochloric acid was added dropwise over 30 minutes with further stirring (the pH at the start of hydrochloric acid dropwise addition was 14, and the pH at the completion was 9). After about 30 minutes, the formed precipitates were suction-filtered, and the precipitates collected by filtration were thoroughly washed with water (until the electrical conductivity of the wash solution fell below 1000 µS) and then dried at 80°C overnight. The collected filter cake was coarsely crushed in a mixer and then calcined at 570°C for 5 hours, thereby giving the intended copper- and aluminum-doped non-porous silica as a slightly bluish white powder.

Incidentally, the amounts of copper chloride as a raw material for doping copper in a non-porous silica, aluminum chloride as a raw material for doping aluminum in a non-porous silica, and water as a solvent used per 1 mol of sodium silicate as a silica raw material were each as follows.
Copper chloride: 0.0201 mol
Aluminum chloride: 0.0475 mol
Water for solution (a) preparation: 48.6 mol
Water for solution (b) preparation: 90.2 mol
In addition, 1.59 mol of hydrochloric acid was used per 1 mol of sodium silicate as a silica raw material.

The copper- and aluminum-doped non-porous silica obtained by the above method had a BET specific surface area of 18.8 m²/g. In addition, the content of copper in the copper- and aluminum-doped non-porous silica was 1.43 wt%, and the aluminum content was 2.03 wt%. The doping of the non-porous silica with copper and aluminum was confirmed using an X-ray photoelectron spectrometer and a transmission electron microscope.

### Production Example 10: Production of Antiviral Aerosol Composition of the Present Invention (No. 10)

In the same manner as in Production Example 1 except that the copper- and aluminum-doped non-porous silica obtained in Production Reference Example 2 was used in place of the copper- and aluminum-doped mesoporous silica obtained in Production Reference Example 1, a coating composition, in which the content of the copper- and aluminum-doped non-porous silica having a median diameter of about 0.5 µm was 5 wt%, the content of the copolymer of vinylpyrrolidone and vinyl acetate was 5 wt%, and the content of ethanol was 90 wt%, was obtained. The obtained coating composition and ethanol were mixed in a weight ratio of 1:4, thereby giving a stock solution in which the content of the copper- and aluminum-doped non-porous silica was 1 wt%, the content of the copolymer of vinylpyrrolidone and vinyl acetate was 1 wt%, and the content of ethanol was 98 wt%. The obtained stock solution and a liquefied petroleum gas as a propellant were filled into an aerosol can in a weight ratio of 6:4, thereby giving an antiviral aerosol composition of the present invention in which the content of the copper- and aluminum-doped non-porous silica was 0.6 wt%, the content of the copolymer of vinylpyrrolidone and vinyl acetate was 0.6 wt%, the content of ethanol was 58.8 wt%, and the content of the liquefied petroleum gas was 40.0 wt%. This antiviral aerosol composition of the present invention had spray deposition properties and stability equal to those of the antiviral aerosol composition of the present invention produced in Production Example **1.** In addition, a coating film formed from this antiviral aerosol composition of the present invention had antiviral action and durability equal to those of the coating film formed from the antiviral aerosol composition of the present invention produced in Production Example **1.**

### Industrial Applicability

The present invention makes it possible to provide an antiviral aerosol composition that has high stability and can be aerosol-applied without causing nozzle clogging or the like, the antiviral aerosol composition being capable of forming a coating film whose copper-doped silica exhibits excellent antiviral properties and which is firmly retained on the surface of an article; a coating film formed from the antiviral aerosol composition; and an article comprising the coating film. In this respect, the present invention is industrially applicable.

## Claims

1. An antiviral aerosol composition comprising:
(1) a copper-doped silica;
(2) a vinylpyrrolidone unit-containing polymer;
(3) an alcohol-based solvent or a mixed solvent of an alcohol-based solvent and water; and
(4) a propellant.

2. The antiviral aerosol composition according to claim 1, wherein the silica is further doped with aluminum.

3. The antiviral aerosol composition according to claim 1, wherein the silica is a porous silica.

4. The antiviral aerosol composition according to claim 1, wherein the pyrrolidone unit-containing polymer is a copolymer of a vinylpyrrolidone unit and a non-vinylpyrrolidone unit.

5. The antiviral aerosol composition according to claim 4, wherein the ratio between the vinylpyrrolidone unit and the non-vinylpyrrolidone unit (vinylpyrrolidone unit:non-vinylpyrrolidone unit) is 2:8 to 8:2.

6. The antiviral aerosol composition according to claim **4,** wherein the copolymer of a vinylpyrrolidone unit and a non-vinylpyrrolidone unit is a copolymer of vinylpyrrolidone and vinyl acetate.

7. The antiviral aerosol composition according to claim **1,** wherein the pyrrolidone unit-containing polymer is polyvinylpyrrolidone.

8. The antiviral aerosol composition according to claim 1, wherein the pyrrolidone unit-containing polymer has a weight average molecular weight of 5,000 to 1,500,000.

9. The antiviral aerosol composition according to claim 1, wherein the alcohol-based solvent is ethanol.

10. The antiviral aerosol composition according to claim 1, wherein the propellant is at least one member selected from the group consisting of a liquefied petroleum gas, a nitrogen gas, a carbon dioxide gas, dimethyl ether, a hydrofluoroolefin, and a hydrofluorocarbon.

11. The antiviral aerosol composition according to claim 1, wherein the contents of the copper-doped silica, the vinylpyrrolidone unit-containing polymer, the alcohol-based solvent or the mixed solvent of an alcohol-based solvent and water, and the propellant in the antiviral aerosol composition, respectively, are as follows: the content of the copper-doped silica is 0.1 to 5 wt%, the content of the vinylpyrrolidone unit-containing polymer is 0.1 to 5 wt%, the content of the alcohol-based solvent or the mixed solvent of an alcohol-based solvent and water is 30 to 90 wt%, and the content of the propellant is 5 to 65 wt%.

12. The antiviral aerosol composition according to claim 1, wherein the weight ratio between the copper-doped silica and the vinylpyrrolidone unit-containing polymer (copper-doped silica: vinylpyrrolidone unit-containing polymer) is 5:95 to 75:25.

13. A method for producing an antiviral aerosol composition comprising a step of mixing a stock solution with (4) a propellant,
the stock solution being obtained by a preparation method comprising a step in which
(1) a copper-doped silica;
(2) a vinylpyrrolidone unit-containing polymer; and
(3) an alcohol-based solvent or a mixed solvent of an alcohol-based solvent and water are housed in a treatment container and ball-milled.

14. A coating film formed from the antiviral aerosol composition according to claim 1.

15. An article comprising the coating film according to claim 11.
